# EUROPEAN PATENT APPLICATION

(11) **EP 2 630 958 A1**
(43) Date of publication of application: **28.08.2013**
(21) Application number: 11834707.9
(22) Date of filing: 17.10.2011
(51) Int. Cl.: A61K 31/194, A61K 33/00, A61K 9/08

(54) **SOLUTIONS FOR BICARBONATE HEMODIALYSIS**

(30) Priority: 18.10.2010 RU 2010143339
(71) Applicant: Obshchestvo s Ogranichennoy Otvetstvennost'yu " NPO "Nephron" Ltd., St. Petersburg 198099 (RU)
(72) Inventor: SMIRNOV, Aleksey Vladimirovich, St.Petersburg 197187 (RU); LAZEBA, Valentina Aleksandrovna, St.Petersburg 199397 (RU); SAPOZHNIKOV, Denis Borisovich, St.Petersburg 192281 (RU)
(74) Representative: Maughan, Sophie Louise
(86) International application number: PCT/RU2011/000822
(87) International publication number: WO 2012/053942

(57) **Abstract**

A concentrated acid component for bicarbonate hemodialysis is proposed, said component comprising a pH-regulating component, where the pH-regulating component comprises hydrochloric acid and succinic acid, and also a kit for producing a component of this type, a diluted acid component and a dialyzing solution based thereon and methods for producing the solutions are proposed.

## Description

### Field of the invention

The invention relates to the field of medicine and concerns solutions for dialysis, employed in acute and chronic renal insufficiency.

### Prior art

In patients with renal insufficiency, the function of the kidneys is replaced by dialysis. The main function of the dialysis solution is to correct the basic parameters of the biochemical composition of the blood of a patient with uremia to normal physiological values. This implies not only removing uremic metabolic products and excess electrolytes, but also supplementing the buffering components of the blood in order to restore the acid-base balance.

In order to achieve these aims, the dialysis solution is prepared such that its chemical composition corresponds to the parameters of normal plasma with certain specific differences. Ions of sodium, potassium, calcium, magnesium and chlorine, and also a buffering component, are normally present in dialysis solution.

The buffering component employed may be acetate, which is metabolized in the body and converted into bicarbonate, this being a natural key component of the main buffering system of the mammal body. A disadvantage of using acetate in dialysis solution is overloading the body with acetate ions during dialysis and a rise in acidosis.

Bicarbonate may also be used directly as the buffering component. The bicarbonate buffering base does not require intermediate metabolism.

However, sodium bicarbonate reacts chemically with salts of calcium and magnesium, with formation of carbonate salts which precipitate out.

In order to resolve this problem, it is customary to prepare dialysis solution with bicarbonate buffer immediately before delivering to the dialyzer, using two pre-prepared components: an acid component which includes salts of Na, Ca, Mg and K, and an alkaline component which includes bicarbonate.

However, even in this case the use of bicarbonate buffer for hemodialysis requires a solution of the problem of preventing the precipitation of calcium carbonate when these two components are mixed.

In standard bicarbonate dialysis solution, a bicarbonate ions content in the range 30-40 mmol/I and a calcium ions content of up to 3.5 mmol/I is permissible. It has been calculated that the pH of this solution must not exceed 7.3 in order to reduce the risk of the calcium carbonate precipitating out. For this purpose, an acid is added to the acid component, this allowing the required pH value of the dialysis solution to be established when the two solutions are mixed.

Conventionally, acetic acid is used in the acid component for bicarbonate hemodialysis.

Attempts to replace acetic acid by citric acid (WO 00/23086, 27.04.2000) or succinic acid (JP 2010-042124, 25.02.2010) are known. However, in both cases the plasma concentration of the acid anion many times exceeds the physiological level, which leads to disturbances of a metabolic character.

In this case, there is the problem of the negative influence of acetate which enters the human body as a result of using such solution. The plasma acetate level during such bicarbonate hemodialysis normally does not exceed 0.1 mmol/l, which is very much lower than during acetate hemodialysis, but is still higher than the normal value.

Furthermore, as before, the fundamental problems of patients on chronic dialysis have not been resolved, such as adequate correction of the acid-base state, a rise ("rebound") in plasma phosphate level after hemodialysis, instability of hemodynamics during the procedure, normalization of amino-acid metabolism, elimination of disorders of protein metabolism, which are a common problem during chronic renal insufficiency, optimization of body weight, correction of metabolic acidosis, renal osteodystrophy, disorder of the phosphorus-calcium-parathyroid hormone balance, and also a fall in the total interleukin-1-inducing potential of the hemodialysis procedure.

There thus exists a need for new and improved solutions for hemodialysis.

### Substance of the invention

According to the present invention, a concentrated acid component for bicarbonate hemodialysis is proposed, comprising a pH-regulating component, where the pH-regulating component comprises hydrochloric acid and succinic acid.

The ratio of hydrochloric acid and succinic acid in such a component may be from 3:1 to 1:3, for example from 2:1 to 1:1, and in particular 2:1.

The concentrated acid component according to the invention may also contain an acid selected from the group comprising citric acid, malic acid, lactic acid and α-ketoglutaric acid or any combination thereof.

The concentrated acid component according to the invention may additionally contain glucose.

Also proposed is a diluted acid component for bicarbonate hemodialysis, comprising a pH-regulating component, where the pH-regulating component comprises hydrochloric acid and succinic acid.

This diluted acid component may be prepared by dilution of the concentrated acid component according to the invention.

In the diluted acid component according to the invention, the pH-regulating component may be present in an amount of 3 mmol/l, wherein the hydrochloric acid is present in the range from 0.1 to 2.9 mmol/l, and the succinic acid in the range from 0.1 to 2.9 mmol/l.

Also proposed is a dialysis solution for bicarbonate hemodialysis, comprising a pH-regulating component, where the pH-regulating component comprises hydrochloric acid and succinic acid.

The dialysis solution according to the invention may be prepared by diluting the concentrated acid component according to the invention and mixing bicarbonate therewith.

In a particular embodiment, the dialysis solution according to the invention may have the following composition, mmol/l:

| | |
|---|---|
| Sodium Na⁺ | 135.0-142.0 |
| Calcium Ca⁺⁺ | 0-1.75 |
| Magnesium Mg⁺⁺ | 0.5-1.0 |
| Potassium K⁺ | 0.3-4.5 |
| Glucose, | 0-11.0 |
| Chlorine Cl⁻ | 136.7-154.9 |
| Hydrogen H⁺ | 2.0-5.0 |
| Succinic acid ion C₄H₄O₄ | 0.1-5.0 |
| Sodium bicarbonate ion HCO₃ or, in particular, | 27.0-40.0 |
| Sodium chloride | 137.3-140.3 |
| Calcium chloride | 1.2-1.75 |
| Magnesium chloride | 0.5-1.0 |
| Potassium chloride | 0.3-4.0 |
| Glucose | 1.0-5.0 |
| Hydrochloric acid | 0.1-2.9 |
| Succinic acid | 0.1-2.9 |
| Sodium bicarbonate | 32.0 |

Also proposed is a kit for bicarbonate hemodialysis comprising electrolytes including a pH-regulating component, where the pH-regulating component comprises hydrochloric acid and succinic acid, wherein the electrolytes may be proved in the dry and/or liquid form.

It is desirable that the hydrochloric acid should be in the liquid form and all remaining components in the dry form.

A kit according to the invention preferably includes the following chemical reagents: sodium chloride NaCl, calcium chloride CaCl₂·2H₂O, magnesium chloride MgCl₂·6H₂O, potassium chloride KCI, hydrochloric acid HCl, succinic acid C₄H₆O₄ and optionally glucose.

A kit according to the invention may additionally comprise an acid selected from the group comprising citric acid, malic acid, lactic acid and α-ketoglutaric acid or any combination thereof.

Also proposed is a method for preparing a concentrated acid component for bicarbonate hemodialysis, wherein the dry chemical reagents for the acid component from the kit according to the invention are dissolved in water and the liquid reagents are added.

Also proposed is a method for preparing a dialysis solution, wherein the concentrated acid component for bicarbonate hemodialysis according to the invention is fed into a hemodialysis unit, where the dilution thereof and mixing with the bicarbonate component are carried out.

In one of the embodiments of the invention, the method for preparing a dialysis solution leads to the preparation of a solution with the following composition, mmol/l:

| | |
|---|---|
| Sodium Na⁺ | 135.0-142.0 |
| Calcium Ca⁺⁺ | 0-1.75 |
| Magnesium Mg⁺⁺ | 0.5-1.0 |
| Potassium K⁺ | 0.3-4.5 |
| Glucose, | 0-11.0 |
| Chlorine Cl⁻ | 136.7-154.9 |
| Hydrogen H⁺ | 2.0-5.0 |
| Succinic acid ion C₄H₄O₄ | 0.1-5.0 |
| Sodium bicarbonate ion HCO₃ or, in particular | 27.0-40.0 |
| Sodium chloride | 137.3-140.3 |
| Calcium chloride | 1.2-1.75 |
| Magnesium chloride | 0.5-1.0 |
| Potassium chloride | 0.3-4.0 |
| Glucose | 1.0-5.0 |
| Hydrochloric acid | 0.1-2.9 |
| Succinic acid | 0.1-2.9 |
| Sodium bicarbonate | 32.0 |

### Detailed description of the invention

At the present time, a hemodialysis procedure is performed with the aid of hemodialysis equipment. Hemodialysis units include devices for supplying blood, devices for preparing and supplying a dialysis solution, and a dialyzer. The dialysis solution is fed into the dialyzer from a reservoir or metering elements in which the solution is prepared immediately before use. Dialysis solution for bicarbonate hemodialysis is prepared from two components, each of which may be stored either in the form of a concentrate or in the form of the dry substance.

The composition of hemodialysis solutions corresponds to human blood in respect of osmolarity, the concentrations of the main ions and the value of the hydrogen ion index (pH). These parameters are taken into account when preparing the formulation of a solution.

It is standard practice for the composition of a dialysis solution to include electrolytes ensuring the presence of the following ions, in mmol/l:

| | |
|---|---|
| Sodium (Na⁺) | 130.0-145.0 |
| Calcium (Ca²⁺) | 0.0-3.5 |
| Magnesium (Mg²⁺) | 0.5-1.0 |
| Potassium (K⁺) | 0.0-4.0 |
| Chlorine (Cl⁻) | 98-124 |
| Bicarbonate (HCO₃⁻) | 27.0-40.0 |
| Hydrogen (H⁺) | 2.0-9.0 |

Sodium is usually supplied in the form of sodium chloride (NaCl), calcium in the form of calcium chloride (CaCl₂·2H₂O), magnesium in the form of magnesium chloride (MgCl₂·6H₂O), and potassium in the form of potassium chloride (KCI), these same salts ensuring that chlorine ion is present, while bicarbonate is supplied in the form of sodium bicarbonate (NaHCO₃). Such substances are standard, although theoretically other salts may be used as components of the solution for bicarbonate hemodialysis, and it is clear to a person skilled in the art what substitution may be made.

The content of each of the components may vary, while the content of each of them has a specific influence on the course of the hemodialysis procedure. Thus, despite the fact that specific ranges are given here, other amounts of the electrolytes may be used in the solution for bicarbonate hemodialysis depending on the individual features of the patient. For example, for the prophylaxis of hyperparathyroidism, the concentration of calcium needs to be carefully balanced in relation to the intake of vitamin D analogues and calcium-containing phosphate-binding preparations.

The composition of the solution for dialysis may also include glucose. However, solutions without glucose may equally well be used. An increase in the osmolarity of the dialysis solution due to glucose facilitates ultrafiltration. The use of a dialysis solution without glucose facilitates the removal of potassium ions from the patient's blood and reduces the level of insulin. If glucose is included, its concentration may be from 0 to 12 g/l, and is usually 11.2 mmol/l.

A dialysis solution may also include additional components, such as insulin, or other substances which it is desirable to use in a specific case.

An acid is a necessary component of a dialysis solution. In the present description, this component of a dialysis solution is designated as a "pH-regulating component", since its purpose is to neutralize bicarbonate ions to such an extent as to prevent the precipitation of deposits when the components for preparing the dialysis solution are mixed immediately before it is used.

The dialysis solution is prepared immediately before use by mixing the "acid component" and the "bicarbonate component". The composition of the acid component includes all the electrolytes with the exception of sodium bicarbonate. The sodium bicarbonate forms a separate component.

During mixing, a small amount (about 4 mmol) of acid reacts with an equimolar amount of bicarbonate with formation of CO₂, which is then converted into carbonic acid. As a result of this, the pH of the dialyzate falls to 7.0-7.3, which reduces the risk of precipitation of the carbonate salts of calcium and magnesium.

The "acid component" and the "bicarbonate component" from which the dialysis solution for bicarbonate hemodialysis is prepared may be in the form of concentrates (known respectively as the "acid component concentrate" and the "bicarbonate component concentrate"). A "dilute acid component" is obtained after dilution of the acid concentrate with water.

At the present time, an acid concentrate with an amount of acetic acid (the pH-regulating component) such that the concentration thereof in the final dialysis solution is 3-4 mmol/I is normally used for bicarbonate hemodialysis. A smaller amount of the acid brings the concentration of calcium carbonate closer to the precipitation point and even a small additional increase in the pH may cause precipitation of the calcium carbonate. Precipitation may also be provoked by the use of old bicarbonate concentrate or excessive losses of carbon dioxide therefrom. In this regard, the situation is complicated when using centralized distribution systems for dialysis solution, in that the prepared bicarbonate dialysis solution must be transported over quite large distances between the central distribution point, at which it is prepared, and the dialysis locations. A higher concentration of acid in the dialysis solution than has been indicated above may be required in order to prevent the precipitation of calcium carbonate in such conditions, i.e. in specific situations the concentration of this component may be up to 5 mmol/l.

The composition of the pH-regulating component may include not only an acid, but also, for example, sodium acetate, which acts as a buffer and stabilizes the pH of the solution.

In certain cases, the concentration of the acid may be reduced to 2 mmol/l.

The concentration of the bicarbonate, which is usually indicated by the manufacturer of concentrates on the canister label, is its final concentration in the dialysis solution prepared with a specified combination of the acid concentrate and the bicarbonate concentrate. This means that the neutralization reaction between hydrogen ions from the acid component and the corresponding number of bicarbonate ions from the bicarbonate component concentrate is taken into account in the calculation.

Some clinics prepare dialysis solution concentrate independently from dry salts, and special apparatus exists for this purpose.

Apparatus for preparing dialysis solution concentrate comprises vessels of varying capacity, in which are mixed chemically pure water (from a water purification system) and weighed amounts of salts in predetermined proportion and volume.

Since standard equipment is used for the dilution of concentrates, it is customary to prepare concentrates which will be subjected to standard dilution. For example, an acid concentrate is mixed with water in a dialysis machine in a standard mixing ratio of 1+34 or 1+44. However, other dilution schemes are, in principle, possible.

Water of dialysis quality is used for dilution.

Mixing of the acid concentrate and bicarbonate with water, and formation of the dialysis solution takes place in a hemodialysis unit (dialysis machine).

Since the term "artificial kidney" may be applied both to the actual hemodialysis unit and also directly to the dialyzer, which is a part thereof, it is specified in this description that "artificial kidney" is to be understood as the hemodialysis unit as a whole.

Standard dilution is to be understood as 1.0 l of liquid acid concentrate, 1.225 l of 8.4% sodium bicarbonate solution and 32.775 l of purified water. A different dilution may however be used.

Modern dialysis machines have a device for connecting a dry bicarbonate cartridge in place of liquid bicarbonate concentrate. In this case, the hydraulic system of the dialysis machine dissolves the bicarbonate cartridge with a metered amount of purified water and feeds this into the mixing system instead of bicarbonate from a canister. In this case, only the acid component (concentrate) is prepared in the liquid state. There are, however, systems which, instead of the liquid acid component, employ special packs with dry salts which allow the composition of the dialysis solution to be strictly individualized and preparation thereof in the liquid form to be entirely avoided.

The "artificial kidney" unit allows a controlled process of blood purification by removal of uremic toxins and regulation of the water-electrolyte balance and the acid-base state to be performed. Controllability of the blood purification procedure is achieved by selecting the blood perfusion and dialyzate parameters in the dialyzer, the rate and volume of ultrafiltration, the mixing proportions of purified water and concentrate, the dialysis temperature, etc.

In modern "artificial kidney" units, the operating conditions are determined and set by the operator before starting a session, depending on the condition of the patient, and may be varied during the entire procedure.

Some sort of organic acid is normally used as the pH-regulating component in solutions for bicarbonate hemodialysis, and thus also in acid component concentrates and kits of dry salts. Acetic acid is conventionally employed, and a reliable substitute for this has not been found, despite the problem of elevated acetate content associated therewith. Although attempts have been made to replace it with another organic acid, particularly citric acid, it is thought that other organic acids will have just the same effect (a plasma level of organic acid anion above the normal). It was also thought that the use of an inorganic acid, such as hydrochloric acid, for example, might lead to extremely low pH values in the acid concentrate.

The inventors of the present invention have unexpectedly found that substituting acetic acid with a mixture of hydrochloric and succinic acids makes it possible to eliminate many of said problems. The acetic acid previously used has the properties of a buffer, which allowed the hydrogen ion concentration to be maintained during the hemodialysis procedure, while hydrochloric acid does not possess buffer properties. It has, however, been found that no uncontrollable rise in hydrogen ion concentration is observed when performing the hemodialysis procedure with a dialysis solution containing a mixture of hydrochloric acid and succinic acid as the pH-regulating component.

Furthermore, the applicant has found that tissue respiration is improved if several different organic acids are used in that part of the pH-regulating component which is represented by organic acid. Without restriction by any particular theory, it may be hypothesized that stimulation of the tricarboxylic acid cycle occurs, the output of energy necessary for ATP synthesis is increased, and oxygen consumption by tissues rises due to enhancement of electron transport in mitochondria.

It was unexpectedly found that a combination of acids according to the invention leads to a reduction in the intensity of acidosis, including cellular acidosis.

The mixture of hydrochloric acid and succinic acid provides a synergistic effect, demonstrating an advantage over the use of each acid separately. The use of these acids separately had disadvantages, which were virtually entirely eliminated on combined use with maintenance of the basic purpose - regulation of the acidity level.

A fall in the level of chronic inflammatory and oxidative stresses, which lie at the root of protein-energy insufficiency and accelerated atherogenesis in patients with terminal uremia, a substantial improvement in liver function, which is particularly important since up to 80% of patients on dialysis suffer from chronic viral hepatitis B and C, and a reduction in the level of phosphates, which is very important for patients with chronic renal insufficiency, were observed in patients receiving treatment by hemodialysis using a solution according to the invention.

Preparation of a hemodialysis solution for bicarbonate hemodialysis according to the invention includes assembling a kit of dry salts, subsequently dissolving these, for example at room temperature, in purified water to form a concentrated acid component for bicarbonate hemodialysis, delivering this component to a hemodialysis unit, and in automated mode diluting the concentrated acid and bicarbonate components in a predetermined ratio.

As a result, a hemodialysis solution is obtained, containing the following components, in mmol/l:

| | |
|---|---|
| Sodium Na⁺ | 135.0-142.0 |
| Calcium Ca⁺⁺ | 0-1.75 |
| Magnesium Mg⁺⁺ | 0.5-1.0 |
| Potassium K⁺ | 0.3-4.5 |
| Glucose, | 0-11.0 |
| Chlorine Cl⁻ | 136.7-154.9 |
| Hydrogen H⁺ | 2.0-5.0 |
| Succinic acid ion C₄H₄O₄ | 0.1-5.0 |
| Sodium bicarbonate ion HCO₃ | 27.0-40.0 |

Each stage of this method can be performed separately and independently of the other stages.

Thus, assembling a kit of dry salts may lead to the preparation of a commercial kit, in which all the components are in the dry form except for those which in normal conditions cannot be provided as dry substances, such as hydrochloric acid.

The concentrated acid component may be prepared from such a kit in appropriate apparatus, filled into canisters and left for storage or sold commercially.

In order to prepare a dialysis solution, the final consumer may use either a concentrated acid component from a canister, or a kit of dry salts from cartridges.

In general, all the products and methods according to the invention may alternatively include, consist of, or essentially consist of any suitable components and stages disclosed in this description or known from the prior art to a person skilled in the art, and also products or methods according to the invention may additionally or alternatively exclude any particular component, or stage, or object which has been used in a product or method known from the prior art, or which is not necessary in order to achieve the technical result of this invention.

### Specific examples of performance of the invention

### EXAMPLES 1-4

Solutions of the following compositions were prepared, mmol/l.

| | N°1 | N°2 | N°3 | N°4 |
|---|---|---|---|---|
| Sodium chloride | 137.3 | 137.3 | 140.3 | 140.3 |
| Calcium chloride | 1.2 | 1.2 | 1.75 | 1.75 |
| Magnesium chloride | 0.5 | 0.5 | 1.0 | 1.0 |
| Potassium chloride | 0.3 | 0.3 | 4.0 | 4.0 |
| Glucose g/l | 5.0 | 5.0 | 1.0 | 1.0 |
| Hydrochloric acid | 2.9 | 0.1 | 2.0 | 2.2 |
| Succinic acid | 0.1 | 2.9 | 1.0 | 0.8 |
| Sodium bicarbonate | 32.0 | 32.0 | 32.0 | 32.0 |

A kit of dry salts (NaCl, CaCl₂.2H₂O, MgCl₂·6H₂O, KCI, glucose, succinic acid) was used to prepare 100 l of concentrated acid component.

The salts from a kit of dry salts were charged into a vessel filled with water purified for hemodialysis, the stirrer was switched on and the solution was stirred until the salts had fully dissolved, then the hydrochloric acid was added. The volume of the resultant solution was then adjusted to 100 l to give a concentrated solution of the acid component. The content of Na⁺ , Ca⁺⁺, Mg⁺⁺, K⁺, Cl⁻, and C₂H₄(COO⁻)₂ ions, and the acidity, pH and osmolarity of the resultant solution were checked. Before feeding the prepared acid component into the "artificial kidney" unit, it was filtered through a filter with a pore size of 100 µm.

The prepared solution and a concentrated solution of the bicarbonate component were fed into the "artificial kidney" unit, in which they were diluted in automated mode in accordance with a preset ratio programme to give solutions of said compositions.

### EXAMPLES 5-8

The same actions are performed as in Example 1 and a solution is prepared with the following content of components, mmol/l:

| | N°5 | N°6 | N°7 | N°8 |
|---|---|---|---|---|
| Sodium chloride | 137.3 | 137.3 | 140.3 | 140.3 |
| Calcium chloride | 1.2 | 1.2 | 1.75 | 1.75 |
| Magnesium chloride | 0.5 | 0.5 | 1.0 | 1.0 |
| Potassium chloride | 0.3 | 0.3 | 4.0 | 4.0 |
| Glucose g/l | 5.0 | 5.0 | 1.0 | 1.0 |
| Hydrochloric acid | 2.0 | 2.0 | 2.0 | 2.0 |
| Succinic acid | 0.5 | 0.5 | 0.2 | 0.1 |
| Lactic acid | 0.5 | | | 0.1 |
| α-ketoglutaric acid | | 0.5 | 0.2 | 0.1 |
| Malic acid | | | 0.1 | 0.1 |
| Citric acid | | | | 0.1 |
| Sodium bicarbonate | 32.0 | 32.0 | 32.0 | 32.0 |

### EXAMPLE 9

Crosssover studies were performed in order to test the effect of the solutions.

A crossover study consisted initially of 2 groups: control (n=39) and experimental (n=51). The two groups did not differ in relation to age, sex, basic initial pathology, length of hemodialysis treatment and nature of comorbidity. Patients were assigned randomly to the groups. Over a period of 6 months, the control group was given sessions of hemodialysis using bicarbonate hemodialysis solution with acetic acid as the pH-regulating component (standard solution), while the experimental group had sessions of hemodialysis using a solution according to the invention.

The dialysis treatment regime was the same in both groups (3 times per week, each of 4.5 hours), and the hemodialysis sessions were performed using the standard method.

Six months after the start of the study, a 3-month "washout" period was applied, during which both groups received hemodialysis treatment using the standard solution.

Thereafter, the groups changed places: the patients of the "control group" (n=39) started to receive hemodialysis sessions with a solution according to the invention, while the "experimental group" remained on hemodialysis using the standard solution. This stage of the study continued for 6 months.

It was found from the results of the study that the use of hemodialysis sessions using a solution according to the invention over a period of 6 months was accompanied by a significant reduction in the blood serum concentration of gamma-glutamyltranspeptidase from 46.62±5.15 to 25.85±4.63 mmol/I (n=90; p<0.002).

Apart from a connection with alcohol consumption, the blood serum gamma-glutamyltranspeptidase level in the general population is associated with a high risk of cardiovascular diseases and strokes. In patients receiving a chronic hemodialysis procedure, a rise in the gamma-glutamyltranspeptidase level is associated with chronic renal insufficiency, concomitant liver diseases, chronic hepatitis and cardiovascular insufficiency. The established fact of a 55% reduction in the level thereof in the blood serum of patients who have been treated by a hemodialysis procedure using the hemodialysis solutions according to the invention can be regarded as favourable in relation to the risk of complications of vascular pathology and mortality, which is more than 50% among patients with terminal renal insufficiency.

Absence of a rebound in the plasma phosphate level was observed in the group of patients who were given hemodialysis with solutions No. 3 and No. 7 according to the invention.

Improvement in the following parameters was also observed:
- ECG characteristics were improved, the BP normalized;
- the urea level fell from 34 mmol/I before dialysis to 25 mmol/I in the experimental group as compared with the control;
- a hypolipidemic effect was observed when using a solution according to the invention: a fall of 2-3 mol/I in cholesterol, LDL cholesterol and Al over 3 months;
- the blood phosphorus level fell;
- the troponin level fell from 0.027 to 0.23 mg/ml;
- the 6-minute-walk test scores improved by 62% after using a solution according to the invention;
- quality of life scores improved.

## Claims

1. A concentrated acid component for bicarbonate hemodialysis, comprising a pH-regulating component, where the pH-regulating component comprises hydrochloric acid and succinic acid.

2. The concentrated acid component according to claim 1, wherein the ratio of hydrochloric acid and succinic acid is from 3:1 to 1:3, for example from 2:1 to 1:1, in particular 2:1.

3. The concentrated acid component according to claim 1, where the pH-regulating component also contains an acid selected from the group comprising citric acid, malic acid, lactic acid and α-ketoglutaric acid or any combination thereof.

4. The concentrated acid component according to any of claims 1-3, which additionally contains glucose.

5. A diluted acid component for bicarbonate hemodialysis, comprising a pH-regulating component, where the pH-regulating component comprises hydrochloric acid and succinic acid.

6. The diluted acid component according to claim 5, which is prepared by dilution of the concentrated acid component according to any of claims 1-4.

7. The diluted acid component according to claim 5, where the pH-regulating component is present in an amount of 3 mmol/l, the hydrochloric acid being present in the range from 0.1 to 2.9 mmol/l, and the succinic acid present in the range from 0.1 to 2.9 mmol/l.

8. A dialysis solution for bicarbonate hemodialysis, comprising a pH-regulating component, where the pH-regulating component comprises hydrochloric acid and succinic acid.

9. The dialysis solution according to claim 8, prepared by diluting the concentrated acid component according to any of claims 1-4 and mixing bicarbonate therewith.

10. The dialysis solution according to claim 8, having the following composition, mmol/l:
| | |
|---|---|
| Sodium Na⁺ | 135.0-142.0 |
| Calcium Ca⁺⁺ | 0-1.75 |
| Magnesium Mg⁺⁺ | 0.5-1.0 |
| Potassium K⁺ | 0.3-4.5 |
| Glucose, | 0-11.0 |
| Chlorine Cl⁻ | 136.7-154.9 |
| Hydrogen H⁺ | 2.0-5.0 |
| Succinic acid ion C₄H₄O₄ | 0.1-5.0 |
| Sodium bicarbonate ion HCO₃ | 27.0-40.0 |

11. The dialysis solution according to claim 8, having the following composition, mmol/l:
| | |
|---|---|
| Sodium chloride | 137.3-140.3 |
| Calcium chloride | 1.2-1.75 |
| Magnesium chloride | 0.5-1.0 |
| Potassium chloride | 0.3-4.0 |
| Glucose | 1.0-5.0 |
| Hydrochloric acid | 0.1-2.9 |
| Succinic acid | 0.1-2.9 |
| Sodium bicarbonate | 32.0 |

12. A kit for bicarbonate hemodialysis, comprising electrolytes, including a pH-regulating component, where the pH-regulating component comprises hydrochloric acid and succinic acid, while the electrolytes may be provided in the dry and/or liquid form.

13. The kit according to claim 12, where the hydrochloric acid is provided in the liquid form, while all remaining components are in the dry form.

14. The kit according to claims 12-13, which includes the following chemical reagents: sodium chloride, calcium chloride, for example CaCl₂.2H₂O, magnesium chloride, for example MgCl₂.6H₂O, potassium chloride, hydrochloric acid, succinic acid and optionally glucose.

15. The kit according to claims 12-13, which additionally comprises an acid selected from the group comprising citric acid, malic acid, lactic acid and α-ketoglutaric acid or any combination thereof.

16. A method for preparing a concentrated acid component for bicarbonate hemodialysis, wherein the dry chemical reagents for the acid component from the kit according to any of claims 12-15 are dissolved in water and the liquid reagents are added.

17. A method for preparing a dialysis solution, wherein the concentrated acid component for bicarbonate hemodialysis according to claims 1-4 is fed into a hemodialysis unit where the dilution thereof and mixing with the bicarbonate component are carried out.

18. The method according to claim 17, which leads to preparation of a solution with the following composition, mmol/l:
| | |
|---|---|
| Sodium Na⁺ | 135.0-142.0 |
| Calcium Ca⁺⁺ | 0-1.75 |
| Magnesium Mg⁺⁺ | 0.5-1.0 |
| Potassium K⁺ | 0.3-4.5 |
| Glucose, | 0-11.0 |
| Chlorine Cl⁻ | 136.7-154.9 |
| Hydrogen H⁺ | 2.0-5.0 |
| Succinic acid ion C₄H₄O₄ | 0.1-5.0 |
| Sodium bicarbonate ion HCO₃ | 27.0-40.0 |

19. The method according to claim 17, which leads to preparation of a solution with the following composition, mmol/l:
| | |
|---|---|
| Sodium chloride | 137.3-140.3 |
| Calcium chloride | 1.2-1.75 |
| Magnesium chloride | 0.5-1.0 |
| Potassium chloride | 0.3-4.0 |
| Glucose | 1.0-5.0 |
| Hydrochloric acid | 0.1-2.9 |
| Succinic acid | 0.1-2,9 |
| Sodium bicarbonate | 32,0 |
